# EUROPÄISCHE PATENTSCHRIFT

(11) **EP 2 280 060 B1**
(45) Veröffentlichungstag und Bekanntmachung des Hinweises auf die Patenterteilung: **19.09.2012**
(21) Anmeldenummer: 10007719.7
(22) Anmeldetag: 26.07.2010
(51) Int. Cl.: C12M 1/107, B01F 7/00, B01F 15/00

(54) **Hub- und Schwenkvorrichtung an einem Führungsmast eines Fermenterbehälters einer Biogasanlage für ein höhenverstellbares Tauchgerät**
Lifting and pivoting device on a guide mast of a fermenter container for a biogas assembly for a height-adjustable submersible device
Dispositif de levage et de basculement sur un mât de guidage d'un récipient de fermentation d'une installation de biogaz pour un appareil de plongée réglable en hauteur

(30) Priorität: 27.07.2009 DE 202009010165 U
(43) Veröffentlichungstag der Anmeldung: 02.02.2011
(73) Patentinhaber: R.E.U.S. Energy GmbH, 83527 Kirchdorf (DE)
(72) Erfinder: Bürger, Adam, 83527 Haag in Obb. (DE)

(56) Entgegenhaltungen:
- DE-A1- 4 120 987
- DE-C1- 19 714 342
- DE-U1- 7 905 739

## Beschreibung

Bei einer bekannten gattungsgemäßen Hub- und Schwenkvorrichtung (DE 195 17 901 C1) an einem Führungsmast eines Fermenterbehälters einer Biogasanlage für ein höhenverstellbares angetriebenes Tauchgerät, insbesondere für ein Tauchrührwerk oder für eine Tauchpumpe ist das Tauchgerät an einem vertikal im Fermenterbehälter angebrachten Führungsmast höhenverschiebbar und unverschwenkbar gehalten, wobei jedoch der Führungsmast selbst um seine Längsachse schwenkbar gelagert ist.

Am Mastoberteil ist eine Höhenverstelleinrichtung angeordnet mit einer betätigbaren Seilwinde zum Auf- und Abwickeln eines Zugseils, das mit dem Tauchgerät und/oder mit einem am Führungsmast verschiebbaren und am Tauchgerät befestigten Führungsschlitten verbunden ist.

Die gasdichte obere Abdeckung des Fermenterbehälters im Bereich des Mastoberteils ist durch eine Abdeckplatte gebildet, wobei das Mastoberteil dicht und verschwenkbar durch die Abdeckplatte geführt ist. Dabei liegt die Seilwinde oberhalb der Abdeckplatte außerhalb des Gasraumes des Fermenterbehälters und das gespannte Zugseil ist von der Seilwinde ausgehend gasdicht durch eine Seildurchführung in den Fermenterinnenraum zum Tauchgerät oder zu dessen Führungsschlitten geführt.

Konkret ist bei der bekannten Hub- und Schwenkvorrichtung die gasdichte Seildurchführung durch die Abdeckplatte punktuell an einer Stelle mit einer Stopfbuchse ausgebildet. Um ein Mitschwenken dieser punktuellen Seildurchführung bei einer Schwenkverstellung des Führungsmastes zu ermöglichen, ist die Abdeckplatte im Bereich des Führungsmastes als Drehteller ausgeführt, durch dessen Mitte die Schwenkachse des Führungsmastes führt. Bei einer Schwenkverstellung des Führungsmastes wird somit auch der Drehteller mit der punktuellen, gegenüber der Schwenkachse versetzten Seildurchführung entsprechend mitgedreht, so dass das gespannte Zugseil im Wesentlichen ungehindert und ohne Rückstellkräfte zwischen der Seilwinde und dem Tauchrührgerät verlaufen kann.

Eine solche Drehtellerlösung ist mechanisch relativ aufwendig und kostenintensiv, da der Drehteller dicht in seinem Drehtellerausschnitt gehalten werden muss. Da der Führungsmast im Drehteller gelagert ist, wirken auf diesen relativ hohe, rührerangeregte Seitenkräfte, die mit hohen Belastungen der Dichtelemente in den Drehtellerführungen aufgenommen werden müssen. Zudem ist hier der Drehteller im Bereich und in der Höhe der Fermenterdecke angeordnet, so dass das Rührgerät zu Wartungszwecken nicht über die Fermenterdecke aus dem Innenbereich des Fermenterbehälters herausgehoben werden kann.

Bei einer weiter bekannten Hub- und Schwenkvorrichtung (DE 197 14 342 C1) wird eine obere Deckenöffnung des Fermenterbehälters von einem Dom dicht übergriffen, wobei in einer oberen Abdeckwand des Doms ein Oberteil eines Führungsmasts schwenkbar und dicht in einer Tauchtasse gehalten ist. Eine betätigbare Seilwinde ist am oberen Mastende außerhalb des Fermentergasraums angeordnet und das Zugseil ist durch den hohlen Führungsmast in den Dorninnenraum und von dort in Umlenkrollen zum höhenverstellbaren Tauchrührwerk geführt. Die hier gezeigte Lösung mit einer Tauchtasse in Verbindung mit der Führungsmastlagerung ist sehr wartungsintensiv. Weiter gehört hier der Mastinnenraum nicht zum Gasraum des Fermenters und die im Fermenterinnenraum angeordnete Gasabdichtung für das Zugseil an der Austrittsstelle aus dem hohlen Führungsmast in den Fermentergasraum ist problematisch. Konkrete Dichtungslösungen sind nicht angegeben. Zudem ist das Einfädeln des Zugseils in den hohlen Führungsmast und durch die Gasabdichtung im Fermenterinnenraum aufwendig, und schwierig. Bei Störungen am Seilzug, insbesondere in Verbindung mit den Umlenkrollen und der Gasabdichtung im Führungsmast sind das Zugseil und die Umlenkrollen nicht offen zugänglich.

Bei einer weiter bekannten ähnlichen Ausführung einer Hub- und Schwenkvorrichtung (DE 197 32 198 C1) ist die Seilwinde im oberen Mastbereich innerhalb des Doms befestigt, so dass für das Zugseil keine gasdichten Durchführungen erforderlich sind. Eine solche Anordnung ist relativ aufwendig in der Herstellung und die Seilwinde mit den Antrieben ist während des normalen Fermenterbetriebs von außen her für Wartungsarbeiten oder zur Behebung von Störungen nicht zugänglich. Bei dieser und der vorstehenden Hub- und Schwenkvorrichtung bei der die Deckenöffnung insgesamt dicht vom Dom überdeckt wird und in den das Tauchrührwerk nach oben gezogen werden kann, ist der Dom relativ groß und teuer. Zudem ist die Zugänglichkeit des hochgezogenen Tauchrührwerks im Dom eingeschränkt.

Aufgabe der Erfindung ist es, eine gattungsgemäße Hub- und Schwenkvorrichtung so auszubilden, dass eine einfache kostengünstige und funktionsfähige Konstruktion möglich wird.

Diese Aufgabe wird mit den Merkmalen des Anspruchs 1 gelöst.

Gemäß Anspruch 1 ist das Mastende am Mastoberteil des Führungsmasts als Lagerzapfenrohr ausgebildet, das gasdicht und verschwenkbar in einem Drehlager in der ortsfesten und unbeweglichen Abdeckplatte gelagert ist. Im Lagerzapfenrohr in der Schwenkachse ist die gasdichte Seildurchführung mit einer Seilabdichtung enthalten, wobei das Zugseil von der Seilwinde durch die in der Schwenkachse liegende Seildurchführung in den einen Teilbereich des Fermentergasraums bildenden Führungsmastinnenraum verläuft und von dort im Bereich des Mastoberteils mit daran angebrachten Umlenkelementen aus dem Führungsmastinnenraum heraus weiter nach unten zum Tauchgerät oder dessen Führungsschlitten geführt ist.

Damit ist eine einfache, gut zugängliche Seilwindenanordnung mit einer einfachen und gut zugänglichen dichten Zugseildurchführung geschaffen, welche eine Verschwenkung des Führungsmasts und damit eine Verschwenkung des Tauchgeräts über weite Schwenkwinkel, insbesondere auch eine Verschwenkung von einer Rührrichtung in die Gegenrührrichtung ohne Behinderung zulässt. Durch die konstruktiv einfache Anordnung der Zugseildurchführung in einem Lagerzapfenrohr ist eine solche Anordnung insbesondere bei einer Seitabdichtung als Stopfbuchse gemäß Anspruch 2 sehr kostengünstig und funktionssicher ausführbar. Auch das Drehlager für das Lagerzapfenrohr in der Abdeckplatte kann gemäß dem bisherigen Stand der Technik kostengünstig hergestellt werden.

In einer konkreten, zweckmäßigen Ausführungsform nach Anspruch 3 ist die Seilwinde auf einer mit dem Lagerzapfenrohr verbunden Tragplatte versetzt zur Schwenkachse angebracht, dergestalt, dass das Zugseil von der Seilablaufstelle an der Seilwinde einfach in das Lagerzapfenrohr einführbar ist. Die Seilwindenbetätigung kann dabei in an sich bekannter Weise von Hand mittels einer Kurbel oder motorisch durchgeführt werden.

Für eine definierte, lagegenaue und gleichmäßige Einführung des Zugseils in das Lagerzapfenrohr, insbesondere auch bei unterschiedlichen Wickelgrößen auf der Seilwinde wird mit Anspruch 4 ein mit dem Lagerzapfenrohr oder der Tragplatte verbundenes Seileinführungselement vorgeschlagen, welches insbesondere als wenigstens eine Einführrolle und/oder als Einführring mit Gleittlächen und/oder Einlaufschrägen ausgeführt sein kann.

Für eine leichtgängige, seilschonende Zugseilumlenkung werden gemäß Anspruch 5 als Umlenkelemente zwei benachbarte Seilrollen mit horizontalen Rollenachsen vorgeschlagen: die obere Seilrolle liegt dabei zumindest teilweise im Führungsmast und die untere Seilrolle ist dazu zumindest teilweise versetzt aus dem Führungsmastinnenraum heraus angeordnet. Dabei verläuft das von oben her kommende Zugseil an der oberen Seilrolle unten und ist von dort an der unteren Seilrolle über diese aus dem Führungsmastinnenraum heraus und weiter nach unten zum Tauchgerät oder dessen Führungsschlitten geführt. Da der Führungsmastinnenraum zum Fermentergasraum gehört, sind für die Seilrollen und deren Lagerungen sowie für die Zugseilführung im Bereich der Seilrollen keine Gasabdichtungen erforderlich, so dass die Anordnung bei einem kompakten Aufbau einfach und kostengünstig herstellbar ist. Zudem können die Seilrollen und die dortige Zugseilführung für Wartungszwecke einfach zugänglich gestaltet werden.

Dazu wird eine besonders bevorzugte konkrete Ausführungsform mit den Merkmalen des Anspruchs 6 vorgeschlagen: dabei ist der Mastoberteil als Mastkopfteil ausgebildet mit einem kastenförmigen Steckschuhbock, an dessen Oberwand das Lagerzapfenrohr vertikal angebracht ist. Die nach unten offene Kastenform enthält die Umlenkelemente, insbesondere in der Art von Seilrollen. Die Kastenform des Mastkopfteils ist dabei so ausgeführt, dass es auf ein quadratisches oder rechteckiges Führungsrohr des Führungsmasts formangepasst aufsteckbar und befestigbar ist. Besonders vorteilhaft kann bei einer solchen Ausführung das Mastkopfteil als Gleichteil in Serie für unterschiedliche Fermenterbehälter hergestellt werden. Die individuelle Anpassung erfolgt dann über die Länge des Führungsrohrs, welches als Stangenware erhältlich ist. Die Befestigung des Mastkopfteils am Führungsrohr kann einfach über lösbare Verschraubungen erfolgen, so dass einerseits eine einfache Montage jedoch auch eine Demontage für Wartungszwecke oder Reparaturen möglich ist. Durch die Ausbildung solcher Mastkopfteile als Gleichteile wird eine erfindungsgemäße Hub- und Schwenkvorrichtung ersichtlich sehr kostengünstig und einfach herstellbar.

In einer konkreten Ausführungsform nach Anspruch 7 ist die Kastenform des Mastkopfteils nach unten und einseitig offen und wird durch die Oberwand, eine Rückwand und zwei, die Lagerstellen für die zwei Seilrollen tragende Seitenwände gebildet. Diese Seitenwände können vorteilhaft in der Art eines Bocks den Bereich des eingesteckten Führungsrohrs überragen, wobei dann in diesem Überstand insbesondere die untere Seilrolle einfach und kompakt gelagert werden kann.

Die vorstehende Hub- und Schwenkvorrichtung ist besonders vorteilhaft in Verbindung mit einem Waftungspodest einsetzbar. Dazu ist nach Anspruch 8 der Führungsmast mit seinem Mastoberteil durch eine Deckenöffnung aus dem Fermenterbehälter nach oben herausgeführt. Die Deckenöffnung ist so groß und die Seilwinde sowie die Umlenkelemente sind so hoch am Mastoberteil angebracht, dass das Tauchgerät aus der Deckenöffnung des Fermenterbehälters heraus bewegbar ist. Im normalen Tauchgerätbetrieb ist die Deckenöffnung durch eine Podestplatte und ein angrenzendes die Ebene der Podestplatte überragendes Mastgehäuse gasdicht abgedeckt, wobei das Mastgehäuse Seitenwände aufweist, nach unten zum Fermenterbehälter offen ist und oben durch eine die Abdeckplatte bildende Gehäuseabdeckung verschlossen ist, welche das Drehlager für das Lagerzapfenrohr mit der Seildurchführung enthält. Eine Seitenwand des Mastgehäuses ist in unmittelbarer Nähe des Führungsmasts angeordnet und als bedarfsweise öffenbare Wartungswand ausgeführt. Im unteren Bereich der öffenbaren Wartungswand grenzt die ebenfalls bedarfsweise öffenbare Podestplatte an, so dass für eine Wartungsbereitstellung des Tauchgeräts die Wartungswand und die Podestplatte öffenbar sind und das Tauchgerät mittels der Seilwinde und dem Zugseil nach oben über die Ebene der geöffneten Podestplatte aus dem Fermenterbehälter aushebbar ist. Dadurch ist das Tauchgerät im Bereich vor der geöffneten Wartungswand frei zugänglich und die Podestplatte kann während einer Wartung unter dem ausgehobenen Tauchgerät wieder angebracht werden, wobei gegebenenfalls verbleibende Spalte gasdicht abdeckbar sind.

Mit den Merkmalen der Ansprüche 9, 10 und 11 sind vorteilhafte, konstruktive Einzelheiten für einen einfachen und kostengünstigen Aufbau beansprucht.

Mit Anspruch 12 sind die Möglichkeiten eines Einbaus in Verbindung mit einer Betondecke und in Verbindung mit einem Foliendach beansprucht.

Anhand einer Zeichnung wird die Erfindung weiter erläutert.

Es zeigen:
- Fig. 1: einen schematischen Teilausschnitt aus einem Fermenterbehälter einer Biogasanlage mit einem an einem Führungsmast höhenver- stellbar gehaltenen Tauchrührgerät mit einer Hub- und Schwenk- vorrichtung;
- Fig. 2: einen vergrößerten Längsschnitt im Bereich des Mastoberteils; und
- Fig. 3: den Längsschnitt aus Fig. 2 um 90° gedreht.

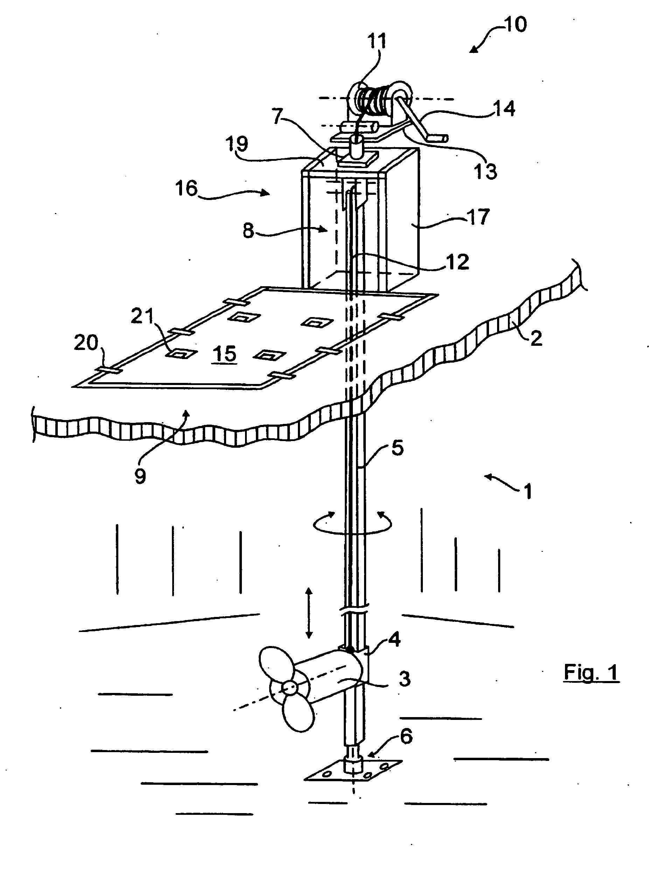

In Fig. 1 ist schematisch ein Teilausschnitt aus einem Fermenterbehälter 1 einer Biogasanlage mit einer Betondecke 2 gezeigt. Ein Tauchrührgerät 3 ist an einem Führungsschlitten 4 befestigt, der an einem Führungsmast 5 in der Art eines Quadratrohrs verschiebbar gehalten ist. Der Führungsmast 5 steht vertikal im Fermenterbehälter 1 und ist in einem unteren Schwenklager 6 und einem oberen gasdichten Drehlager 7 schwenkbar gehalten. Im Bereich der Lager 6, 7 ist am Quadratrohr des Führungsmasts 5 jeweils unmittelbar oder mittelbar ein zylindrischer Lagerzapfen endseitig angebracht.

Der Führungsmast 5 ist mit einem Mastoberteil 8 durch eine rechteckige Deckenöffnung 9 aus dem Fermenterbehälter 1 nach oben herausgeführt.

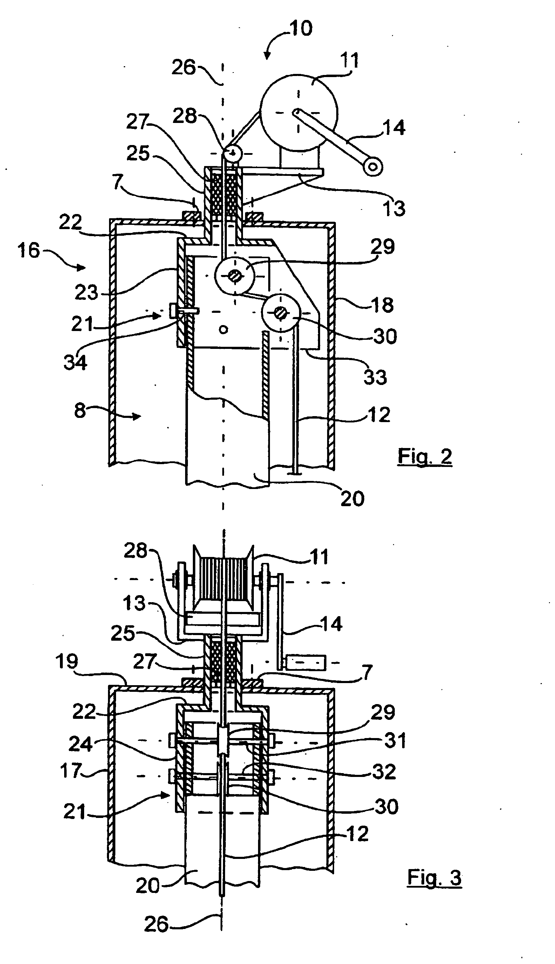

Im oberen Bereich des Mästoberteils 8 ist eine Höhenverstelleinrichtung 10 angebracht, die aus einer betätigbaren Seilwinde 11 mit einem zugeordneten Zugseil 12 besteht, welches für eine Höhenverstellung mit dem Führungsschlitten 4 und damit mit dem Tauchrührwerk 3 verbunden ist. Die Seilwinde 11 ist auf einer mit dem Mastoberteil 8 verschwenkbaren Tragplatte 13 gehalten und mit einer Kurbel 14 betätigbar. Wesentlich ist, dass die Seilwinde 11 oben am Führungsmast 5 angeordnet ist und damit mit diesem mitverschwenkt und dass das Zugseil am Führungsmast 5 entlang nach unten zum Tauchrührwerk 3 und/oder zum Führungsschlitten 4 geführt ist, was detailliert anhand der Fig. 2, 3 erläutert wird. (Eine Energieleitung, insbesondere ein Elektrokabel zum Tauchrührwerk 3 ist der Übersichttichkeit halber nicht eingezeichnet.) Mit der Seilwindenanordnung, gegebenenfalls in Verbindung mit einem Hebel oder einer weiteren Kurbel oder einem Stellmotor kann zur Verstellung der Rührrichtung des Tauchrührwerks 3 eine Schwenkverstellung des Führungsmasts 5 durchgeführt werden.

Die Deckenöffnung 9 ist im normalen Tauchgerätbetrieb durch eine Podestplatte 15 und durch ein angrenzendes die Ebene der Podestplatte 15 überragendes Mastgehäuse 16 dicht abgedeckt. Das Mastgehäuse 16 weist Seitenwände 17 auf, ist nach unten zum Fermenterbehälter 1 offen und durch eine obere Gehäuseabdeckung als Abdeckplatte 19 verschlossen. In dieser Abdeckplatte 19 ist mittig das gasdichte Drehlager 7 für den Mastoberteil 8 angebracht. Der Führungsmast 5 ist mit seinem Mastoberteil 8 im mittleren Bereich einer Rechteckschmalseite der rechtwinkligen Deckenöffnung 9 angeordnet, wo auch das Mastgehäuse 16 liegt. Die zur angrenzenden Podestplattte 15 hinweisende Seitenwand des Mastgehäuses 16 ist als bedarfsweise öffenbare, funktionsmäßig jedoch dicht verschlossene Wartungswand 18 ausgeführt und liegt in unmittelbarer Nähe angrenzend an den Mastoberteil 8, so dass nach einem Öffnen der Wartungswand 8 unmittelbar vor dem Mastoberteil 8 ein frei zugänglicher Raum zur Verfügung steht.

Das Mastgehäuse 16 ist durch ein (nicht dargestelltes) Rahmengestell gebildet und mit den Seitenwänden 17 und der Abdeckplatte 19 beplankt, wobei die Wartungswand 18 bedarfsweise öffenbar ist. Das Mastgehäuse 16 kann bei Bedarf gegebenenfalls durch Stützstreben an der Betondecke 2 abgestützt werden. Die Podestplatte 15 kann in einen Winkelrahmen eingelegt sein und mit Schneliverschlüssen 20 befestigt und bedarfsweise mittels aufklappbarer Handgriffe 21 ausgehoben und entfernt werden.

Die Höhenverstelleinrichtung 10 als Hub- und Schwenkvorrichtung liegt ersichtlich oberhalb der Abdeckplatte 19 außerhalb des Fermentergasraums. Der Mastoberteil 8 ist an seinem Drehlager 7 gasdicht und verschwenkbar durch die Abdeckplatte 19 geführt. Das von der Seilwinde 11 nach unten in den Fermentergasraum führende Zugseil 12 ist gasdicht geführt, wobei die Anordnung und Funktion anhand der Fig. 2 und 3 nachfolgend erläutert werden:

In den korrenspondierenden Schnittdarstellungen der Fig. 2 und 3 ist der Bereich am Mastoberteil 8 beziehungsweise am Mastgehäuse 16 mit der Höhenverstelleinrichtung 10 in Verbindung mit der Mastverschwenkung dargestellt. Am Mastgehäuse 16 sind die Seitenwände 17, die abnehmbare Wartungswand 18 und die obere Abdeckplatte 19 ersichtlich. Der Führungsmast 5 besteht aus einem langen quadratischen Führungsrohr 20, auf das im Bereich des Mastoberteils 8 ein Mastkopfteil 21 aufgesteckt ist. Der Mastkopfteil 21 ist als kastenförmiger Steckschuhbock ausgebildet mit einer Oberwand 22, einer Rückwand 23 und Seitenwänden 24. Gegenüberliegend zur Rückwand 23 ist der Mastkopfteil 21 ebenso wie nach unten offen.

Auf der Oberwand 22 des Mastkopfteils 21 ist ein vertikales Lagerzapfenrohr 25 angebracht, das gasdicht und verschwenkbar im Drehlager 7 an der Abdeckplatte 19 des Mastgehäuses 16 gelagert ist. In Verbindung mit dem unteren Schwenklager 6 ist somit der Führungsmast 5 um seine zentrale Schwenkachse 26 verschwenkbar.

Mit dem Lagerzapfenrohr 5 mitverschwenkend ist die horizontale Tragplatte 13 verbunden, auf der versetzt zur Schwenkachse 26 die Seilwinde 11 mit seiner Kurbel 14 angeordnet und gehalten ist. Im Lagerzapfenrohr 25 in der Schwenkachse 26 ist eine gasdichte Seildurchführung 27, hier in der Art einer Stopfbuchse enthalten, durch die das von der Seilwinde 11 kommende und durch eine Einführrolle 28 gelenkte Zugseil 12 nach unten in den Führungsmastinnenraum beziehungsweise in den Innenraum des Mastkopfteils 21 und damit in den Fermentergasraum geführt ist.

Im Mastkopfteil 21 sind als Seilumlenkelemente zwei Seilrollen 29, 30 mit zugeordneten, an den Seitenwänden 24 gehaltenen Rollenachsen 31, 32 angeordnet. Die obere Seilrolle 29 liegt mit ihrer Einlaufseite unterhalb der Seildurchführung 27, während die etwas darunter liegende untere Seilrolle 30 zur offenen Seite des Mastkopfteils 21 hin versetzt angeordnet ist, wozu die Seitenwände 24 gegenüber dem Führungsrohr 20 einen Überstand 33 als Lagerbock für die untere Seilrolle 30 aufweisen. Ersichtlich ist das Zugseil 12 an der oberen Seilrolle 29 von unten her und an der unteren Seilrolle 30 über diese aus dem Innenraum des Mastkopfteils 21 beziehungsweise des Führungsmasts 5 heraus vor diesen und weiter nach unten zum Eine stabile Verbindung zwischen dem Führungsrohr 20 und dem Mastkopfteil 21 ist zusätzlich zur formschlüssigen Verbindung mit Verschraubungen 24 hergestellt.

## Patentansprüche

1. Hub- und Schwenkvorrichtung an einem Führungsmast eines Fermenterbehälters einer Biogasanlage für ein höhenverstellbares Tauchgerät, wobei
das Tauchgerät (3) an einem vertikal im Fermenterbehälter (1) angebrachten Führungsmast (5) höhenverschiebbar und unverschwenkbar gehalten ist und der Führungsmast (5) um seine Längsachse (26) schwenkbar gelagert ist,
am Mastoberteil (8) eine Höhenverstelleinrichtung (10) angeordnet ist mit einer betätigbaren Seilwinde (11) zum Auf- und Abwickeln eines Zugseils (12), das mit dem Tauchgerät (3) und/oder mit einem am Führungsmast (5) verschiebbaren und am Tauchgerät (3) befestigten Führungsschlitten (4) verbunden ist,
die gasdichte obere Abdeckung des Fermenterbehälters (1) im Bereich des Mastoberteils (8) durch eine Abdeckplatte (19) gebildet ist, wobei das Mastoberteil (8) dicht und verschwenkbar durch die Abdeckplatte (19) geführt ist, so dass die Seilwinde (11) oberhalb der Abdeckplatte (19) außerhalb des Gasraums des Fermenterbehälters (1) liegt und das gespannte Zugseil (12) von der Seilwinde (11) gasdicht durch eine Seildurchführung in den Fermenterinnenraum zum Tauchgerät (3) oder dessen Führungsschlitten (4) geführt ist,
**dadurch gekennzeichnet,**
**dass** das Mastende am Mastoberteil (8) des Führungsmasts (5) als Lagerzapfenrohr (25) ausgebildet ist, das gasdicht und verschwenkbar in einem Drehlager (7) in der ortsfesten und unbeweglichen Abdeckplatte (19) gelagert ist,
**dass** im Lagerzapfenrohr (25) in der Schwenkachse (26) die gasdichte Seildurchführung (27) mit einer Seilabdichtung enthalten ist, wobei das Zugseil (12) von der Seilwinde (11) durch die in der Schwenkachse (26) liegende Seildurchführung (27) in den einen Teilbereich des Fermentergasraums bildenden Führungsmastinnenraum verläuft und von dort im Bereich des Mastoberteils (8) mit daran angebrachten Umlenkelementen (29, 30) aus dem Führungsmastinnenraum heraus weiter nach unten zum Tauchgerät (3) oder dessen Führungsschlitten (4) geführt ist.

2. Hub- und Schwenkvorrichtung nach Anspruch 1, **dadurch gekennzeichnet, dass** die Seildurchführung (27) als Stopfbuchse ausgebildet ist.

3. Hub- und Schwenkvorrichtung nach Anspruch 1 oder Anspruch 2, **dadurch gekennzeichnet, dass** die Seilwinde (11) auf einer mit dem Lagerzapfenrohr (25) verbundenen Tragplatte (13) versetzt zur Schwenkachse (26) angebracht ist und von Hand oder motorisch betätigbar ist.

4. Hub- und Schwenkvorrichtung nach einem der Ansprüche 1 bis 3, dalurch gekennzeichnet, dass über dem Lagerzapfenrohr (25) wenigstens ein Seileinführungselement, insbesondere eine Einführrolle (28) und/oder ein Einführring für eine definierte, lagegenaue Seileinführung in das Lagerzapfenrohr (25) und die Seilabdichtung angeordnet ist.

5. Hub- und Schwenkvorrichtung nach einem der Ansprüche 1 bis 4, **dadurch gekennzeichnet, dass** als Umlenkelemente zwei benachbarte Seilrollen (29, 30) mit horizontalen Rollenachsen (31, 32) vorgesehen sind, von denen eine obere Seilrolle (29) zumindest teilweise im Führungsmastinnenraum liegt und eine untere Seilrolle (30) dazu zumindest teilweise versetzt aus dem Führungsmastinnenraum heraus angeordnet ist, wobei das Zugseil (12) an der oberen Seilrolle (29) von unten her und an der unteren Seilrolle (30) über diese aus dem Führungsmastinnenraum heraus weiter nach unten geführt ist.

6. Hub- und Schwenkvorrichtung nach einem der Ansprüche 1 bis 5, **dadurch gekennzeichnet,**
**dass** der Mastoberteil als Mastkopfteil (21) ausgebildet ist mit einem kastenförmigen Steckschuhbock, an dessen Oberwand (22) das Lagerzapfenrohr (25) vertikal angebracht ist, wobei
die nach unten offene Kastenform die Umlenkelemente, insbesondere die beiden Seilrollen (29, 30) enthält, und
das Mastkopfteil (21) auf ein quadratisches oder rechteckiges Führungsrohr (20) des Führungsmasts (5) formangepasst aufsteckbar und befestigbar ist.

7. Hub- und Schwenkvorrichtung nach Anspruch 6, **dadurch gekennzeichnet, dass** die Kastenform nach unten einseitig offen ist und durch die Oberwand (22), eine Rückwand (23) und zwei die Lagerstellen für die zwei Seilrollen (29, 30) tragende Seitenwände (24) gebildet ist, welche den Bereich des Führungsrohrs (25) überragen und im Überstand (33) insbesondere die untere Seilrolle (30) gelagert ist.

8. Hub- und Schwenkvorrichtung nach einem der Ansprüche 1 bis 7, **dadurch gekennzeichnet,**
**dass** der Führungsmast (5) mit seinem Mastoberteil (8) durch eine Deckenöffnung (9) aus dem Fermenterbehälter (1) nach oben herausgeführt ist,
**dass** die Deckenöffnung (9) so groß ist und die Seilwinde (11) sowie die Umlenkelemente (29, 30) so hoch am Mastoberteil (8) angebracht sind, dass das Tauchgerät (3) aus der Deckenöffnung (9) des Fermenterbehälters (1) herausziehbar ist,
**dass** im normalen Tauchgerätbetrieb die Deckenöffnung (9) durch eine Podestplatte (15) und ein angrenzendes, die Ebene der Podestplatte (15) überragendes Mastgehäuse (16) gasdicht abgedeckt ist, wobei
das Mastgehäuse (16) Seitenwände (17) aufweist, nach unten zum Fermenterbehälter (1) offen ist und oben durch eine die Abdeckplatte (19) bildende Gehäuseabdeckung verschlossen ist, die das Drehlager (7) für das Lagerzapfenrohr (25) mit der Seildurchführung (27) enthält,
**dass** zumindest eine Seitenwand (17) des Mastgehäuses (16) in unmittelbarer Nähe des Führungsmastes (5) angeordnet ist und als bedarfsweise öffenbare Wartungswand (18) ausgeführt ist,
**dass** im unteren Bereich der öffenbaren Wartungswand (18) die ebenfalls bedarfsweise öffenbare Podestplatte (15) angrenzt, so dass für eine Wartungsbereitstellung des Tauchgerätes (3), die Wartungswand (18) und die Podestplatte (15) öffenbar sind und das Tauchgerät (3) mittels der Seilwinde (11) und dem Zugseil (12) nach oben über die Ebene der geöffneten Podestplatte (15) aus dem Fermenterbehälter (1) aushebbar ist, wobei das Tauchgerät (3) im Bereich vor der geöffneten Wartungswand (18) frei zugänglich ist und anschließend die Podestplatte (15) unter dem ausgehobenen Tauchgerät (3) wieder anbringbar und gegebenenfalls verbleibende Spalte gasdicht abdeckbar sind.

9. Hub- und Schwenkvorrichtung nach Anspruch 8, **dadurch gekennzeichnet, dass** die Deckenöffnung (9) etwa rechteckig ist und der Führungsmast (5) bzw. der Mastoberteil (8) im mittleren Bereich einer Rechteckschmalseite angeordnet ist, und
dass das Mastgehäuse (16) im mittleren Bereich dieser Rechteckschmalseite angeordnet ist oder sich als schmales Mastgehäuse über den ganzen Bereich dieser Rechteckschmalseite bis jeweils zu den gegenüberliegenden Rechtecklängsseiten erstreckt.

10. Hub- und Schwenkvorrichtung nach Anspruch 8 oder 9, **dadurch gekennzeichnet, dass** das Mastgehäuse (16) durch ein Rahmengestell gebildet ist mit einer Beplankung durch die Seitenwände (17), von denen wenigstens die Wartungswand (18) öffenbar ist, und durch die Abdeckplatte (19), wobei bedarfsweise Stützstreben unmittelbar oder mittelbar mit dem Rahmengestell verbindbar sind.

11. Hub- und Schwenkvorrichtung nach einem der Ansprüche 8 bis 10, **dadurch gekennzeichnet, dass** die Podestplatte (15) und die Wartungswand (18) zusammen einstückig oder mehrteilig ausgeführt sind.

12. Hub und Schwenkvorrichtung nach einem der Ansprüche 8 bis 11, **dadurch gekennzeichnet, dass** die Deckenöffnung (9) in einer horizontalen Fermenterbehälterdecke, insbesondere einer Betondecke oder in einem geneigt verlaufenden Foliendach liegt und das Mastgehäuse (16) über die Deckenöffnung (9) nach oben abragt.

## Claims

1. A lifting and pivoting apparatus located on a guide mast of a fermentation tank of a biogas plant and intended for a height-adjustable submersible unit, wherein
the submersible unit (3) is retained such that it can be displaced in height, but cannot be pivoted, on a guide mast (5) fixed vertically in the fermentation tank (1), and the guide mast (5) is mounted such that it can be pivoted around its longitudinal axis (26),
a height-adjusting device (10) is arranged on the upper part (8) of the mast, having an actuable cable winch (11) for winding up and unwinding a pulling cable (12) which is connected with the submersible unit (3) and/or with a guide carriage (4) fixed on the submersible unit (3) and can be displaced along the guide mast (5),
the gas-tight upper covering of the fermentation tank (1), in the region of the upper part (8) of the mast, is formed by a covering plate (19), wherein the upper part (8) of the mast is guided in a sealed and pivotable manner through the covering plate (19)such that the cable winch (11) is located above the covering plate (19), outside the gas chamber of the fermentation tank (1), and the tensioned pulling cable (12) is guided in a gas-tight manner from the cable winch (11) through a cable leadthrough into the interior of the fermenter to the submersible unit (3) or to the guide carriage (4) thereof,
**characterized**
**in that** the end of the guide mast (5) at the upper part (8) of the latter is designed as a bearing-journal tube (25) which is mounted in a gas-tight and pivotable manner in a rotary bearing (7) in the location-fixed and non-movable covering plate (19),
**in that** the bearing-journal tube (25), along the pivot axis (26), contains the gas-tight cable leadthrough (27) with a cable sealing, wherein the pulling cable (12) runs from the cable winch (11) through the cable leadthrough (27) which is located along the pivot axis (26) into the guide-mast interior which forms a sub-region of the gas chamber of the fermenter and, from there, in the region of the upper part (8) of the mast and by way of deflecting elements (29, 30) fixed thereon, is guided further downward, out of the guide-mast interior, to the submersible unit (3) or to the guide carriage (4) thereof.

2. The lifting and pivoting apparatus according to claim 1, **characterized in that** the cable leadthrough (27) is designed as a gland.

3. The lifting and pivoting apparatus according to claim 1 or claim 2, **characterized in that** the cable winch (11) is fixed, in a manner offset in relation to the pivot axis (26), on a carrying plate (13) connected with the bearing-journal tube (25), and can be actuated manually or by a motor.

4. The lifting and pivoting apparatus according to any one of claims 1 to 3, **characterized in that** at least one cable-introduction element, in particular an introduction roller (28) and/or an introduction ring intended for a defined, precisely positioned cable introduction into the bearing-journal tube (25) and the cable-sealing means is arranged above the bearing-journal tube (25).

5. The lifting and pivoting apparatus according to any one of claims 1 to 4, **characterized in that** the deflecting elements provided are two adjacent cable rollers (29, 30) which have horizontal roller axes (31, 32) and of which an upper cable roller (29) is located at least in part within the guide-mast interior and a lower cable roller (30) is offset at least in part in relation to the upper cable roller in the direction out of the guide-mast interior, wherein the pulling cable (12) is guided from beneath on the upper cable roller (29) and on the lower cable roller (30) over the latter and further downward in the direction out of the guide-mast interior.

6. The lifting and pivoting apparatus according to any one of claims 1 to 5, **characterized**
**in that** the upper part of the mast is designed as a mast head part (21) with a box-formed plug-in rack, on the upper wall (22) of which the bearing-journal tube (25) is fixed vertically, wherein
the downwardly open box form contains the deflecting elements, in particular the two cable rollers (29, 30), and
the mast head part (21) can be plugged and fastened in a form-fitting manner onto a square or rectangular guide tube (20) of the guide mast (5).

7. The lifting and pivoting apparatus according to claim 6, **characterized in that** the box form is open on one side in the downward direction and is formed by the upper wall (22), a rear wall (23) and two side walls (24), which carry the bearing locations for the two cable rollers (29, 30) and project beyond the region of the guide tube (25), and in particular the lower cable roller (30) is mounted in the projection (33).

8. The lifting and pivoting apparatus according to any one of claims 1 to 7, **characterized**
**in that** the guide mast (5) has its upper part (8) guided upwards through a ceiling opening (9) out of the fermentation tank (1),
**in that** the ceiling opening (9) is such large, and the cable winch (11) and the deflecting elements (29, 30) are fitted such high on the upper part (8) of the mast that the submersible unit (3) can be pulled out of the ceiling opening (9) of the fermentation tank (1),
**in that**, during normal operation of the submersible unit, the ceiling opening (9) is covered in a gas-tight manner by a platform panel (15) and an adjacent mast housing (16), which projects beyond the plane of the platform panel (15), wherein
the mast housing (16) has side walls (17), is open in the downward direction towards the fermentation tank (1) and is closed at the top by a housing covering forming the covering plate (19) and containing the rotary bearing (7) for the bearing-journal tube (25) with the cable leadthrough (27),
**in that** at least one side wall (17) of the mast housing (16) is arranged in the immediate vicinity of the guide mast (5) and is configured as a maintenance wall (18) which can be opened if required,
**in that** the platform panel (15), which can likewise be opened if required, is adjacent to the lower region of the openable maintenance wall (18) such that, in order to provide maintenance on the submersible unit (3), the maintenance wall (18) and the platform panel (15) can be opened and the submersible unit (3) can be lifted out of the fermentation tank (1) in the upward direction, above the plane of the opened platform panel (15), by means of the cable winch (11) and the pulling cable (12), wherein the submersible unit (3) is freely accessible in the region in front of the opened maintenance wall (18), and then the platform panel (15) can be re-fixed beneath the lifted-out submersible unit (3), and any gaps which may still be remaining can be covered in a gas-tight manner.

9. The lifting and pivoting apparatus according to claim 8, **characterized in that** the ceiling opening (9) is approximately rectangular, and the guide mast (5) or the upper part (8) of the mast is arranged in the central region of a narrow side of the rectangle, and
**in that** the mast housing (16) is arranged in the central region of this narrow side of the rectangle or, as a narrow mast housing, extends over the entire region of this narrow side of the rectangle up to each of the opposite longitudinal sides of the rectangle.

10. The lifting and pivoting apparatus according to claim 8 or 9, **characterized in that** the mast housing (16) is formed by a framework with covering provided by the side walls (17), of which at least the maintenance wall (18) can be opened, and by the covering plate (19), wherein, if appropriate, supporting straps can be connected directly or indirectly with the framework.

11. The lifting and pivoting apparatus according to any one of claims 8 to 10, **characterized in that** the platform panel (15) and the maintenance wall (18) are configured together in one piece or in a number of parts.

12. The lifting and pivoting apparatus according to any one of claims 8 to 11, **characterized in that** the ceiling opening (9) is located in a horizontal fermentation tank ceiling, in particular a concrete ceiling or in an inclined sheet-material roof, and the mast housing (16) projects upwards beyond the ceiling opening (9).

## Revendications

1. Un dispositif de levage et de basculement sur un mât de guidage d'un récipient de fermentation d'une installation de biogaz pour un appareil de plongée réglable en hauteur, dans lequel
l'appareil (3) de plongée est maintenu de façon déplaçable en hauteur et non basculante sur un mât (5) de guidage installé verticalement dans le récipient (1) de fermentation et le mât (5) de guidage est monté de façon pivotante autour de son axe (26) longitudinal,
un dispositif (10) de déplacement en hauteur est agencé sur la partie (8) supérieure de mât avec un treuil (11) à câble actionnable pour enrouler et dérouler un câble (12) de traction, qui est relié à l'appareil (3) de plongée et/ou à un chariot (4) de guidage déplaçable sur le mât (5) de guidage et fixé à l'appareil (3) de plongée,
le couvercle supérieur étanche au gaz du récipient (1) de fermentation est formé par une plaque (19) de couverture dans la région de la partie (8) supérieure de mât, dans lequel la partie (8) supérieure de mât est guidée de façon étanche et basculante à travers la plaque (19) de couverture, de telle manière que le treuil (11) à câble soit situé au-dessus de la plaque (19) de couverture à l'extérieur de la chambre à gaz du récipient (1) de fermentation et que le câble (12) de traction tendu soit guidé à partir du treuil (11) à câble de façon étanche au gaz à travers un passage de câble dans la chambre intérieure du récipient de fermentation jusqu'à l'appareil (3) de plongée ou jusqu'au chariot (4) de guidage de celui-ci,
**caractérisé en ce que** l'extrémité du mât à la partie (8) supérieure de mât du mât (5) de guidage se présente sous la forme d'un tube (25) formant tourillon, qui est monté de façon étanche au gaz et basculante dans un palier (7) de rotation dans la plaque (19) de couverture stationnaire et immobile,
**en ce que** le passage (27) de câble étanche au gaz est contenu, avec un joint d'étanchéité de câble, dans le tube (25) formant tourillon sur l'axe (26) de pivotement, dans lequel le câble (12) de traction s'étend du treuil (11) à câble à travers le passage (27) de câble situé sur l'axe (26) de pivotement jusque dans la chambre intérieure du mât de guidage formant une région partielle de la chambre à gaz du récipient de fermentation, et est guidé de là, dans la région de la partie (8) supérieure de mât, avec des éléments (29, 30) de déviation agencés sur celle-ci, hors de la chambre intérieure du mât de guidage ensuite vers le bas jusqu'à l'appareil (3) de plongée ou jusqu'au chariot (4) de guidage de celui-ci.

2. Le dispositif de levage et de basculement selon la revendication 1, **caractérisé en ce que** le passage (27) de câble se présente sous la forme d'une calotte d'obturation.

3. Le dispositif de levage et de basculement selon la revendication 1 ou 2, **caractérisé en ce que** le treuil (11) à câble est disposé sur une plaque (13) de support assemblée au tube (25) formant tourillon en position décalée par rapport à l'axe (26) de pivotement et peut être actionné à la main ou par un moteur.

4. Le dispositif de levage et de basculement selon l'une quelconque des revendications 1 à 3, **caractérisé en ce que** au moins un élément d'introduction de câble, en particulier un galet (28) d'introduction et/ou un anneau d'introduction, pour une introduction définie du câble en position précise dans le tube (25) formant tourillon et le joint d'étanchéité de câble se trouve au-dessus du tube formant (25) tourillon.

5. Le dispositif de levage et de basculement selon l'une quelconque des revendications 1 à 4, **caractérisé en ce qu'**il est prévu comme éléments de déviation deux galets (29, 30) à câble voisins avec des axes (31, 32) de galet horizontaux, parmi lesquels le galet (29) à câble supérieur est situé au moins en partie dans la chambre intérieure du mât de guidage et le galet (30) à câble inférieur est disposé de façon au moins partiellement décalée par rapport au galet (29) à câble supérieur hors de la chambre intérieure du mât de guidage, dans lequel le câble (12) de traction est guidé par le bas sur le galet (29) à câble supérieur et au-dessus sur le galet (30) à câble inférieur hors de la chambre intérieure du mât de guidage puis vers le bas.

6. Le dispositif de levage et de basculement selon l'une quelconque des revendications 1 à 5, **caractérisé en ce que** la partie (8) supérieure de mât se présente sous la forme d'une partie (21) de tête de mât avec un chapeau à emboîter en forme de caisson, sur la paroi (22) supérieure duquel le tube (25) formant tourillon est agencé verticalement, dans lequel
la forme de caisson ouverte vers le bas contient les éléments de déviation, en particulier les deux galets (29, 30) à câble,
et la partie (21) de tête de mât peut être engagée et fixée sur un tube (20) de guidage carré ou rectangulaire du mât (5) de guidage de forme adaptée.

7. Le dispositif de levage et de basculement selon la revendication 6, **caractérisé en ce que** la forme de caisson est ouverte d'un côté vers le bas et est formée par la paroi (22) supérieure, une paroi (23) arrière et deux parois (24) latérales portant les points d'appui pour les deux galets (29, 30) à câble, lesquelles dépassent la région du tube (25) de guidage et où notamment le galet (30) à câble inférieur se repose dans la partie (33) en dépassement.

8. Le dispositif de levage et de basculement selon l'une quelconque des revendications 1 à 7,
**caractérisé en ce que** le mât (5) de guidage est guidé avec sa partie (8) supérieure de mât vers le haut hors du récipient (1) de fermentation à travers une ouverture (9) de plafond,
**en ce que** l'ouverture (9) de plafond a une grandeur telle et le treuil (11) à câble ainsi que les éléments (29, 30) de déviation sont disposés sur la partie (8) supérieure de mât à une hauteur telle que l'appareil (3) de plongée puisse être extrait hors de l'ouverture (9) de plafond du récipient (1) de fermentation,
**en ce qu'**en fonctionnement normal de l'appareil de plongée l'ouverture (9) de plafond est recouverte de façon étanche au gaz par une plaque (15) de socle et une caisse (16) de mât adjacente et dépassant le plan de la plaque (15) de socle, dans lequel la caisse (16) de mât présente des parois (17) latérales, est ouverte vers le bas vers le récipient (1) de fermentation et est fermée vers le haut par un couvercle de caisse formant la plaque (19) de couverture, qui contient le palier (7) de rotation pour le tube (25) formant tourillon avec le passage (27) de câble,
**en ce qu'**au moins une des parois (17) latérales de la caisse (16) de mât est disposée à proximité immédiate du mât (5) de guidage et est réalisée sous la forme d'une paroi (18) d'entretien qui peut être ouverte au besoin,
**en ce que** la plaque (15) de socle pouvant également être ouverte au besoin est adjacente dans la région inférieure de la paroi (18) d'entretien pouvant être ouverte, de telle manière que, pour soumettre l'appareil (3) de plongée à un entretien, la paroi (18) d'entretien et la plaque (15) de socle puissent être ouvertes et que l'appareil (3) de plongée puisse être soulevé vers le haut hors du récipient (1) de fermentation, au-dessus du plan de la plaque (15) de socle ouverte, au moyen du treuil (11) à câble et du câble (12) de traction, dans lequel l'appareil (3) de plongée est librement accessible dans la région située devant la paroi (18) d'entretien ouverte et la plaque (15) de socle peut ensuite être de nouveau placée en dessous de l'appareil (3) de plongée soulevé et les fentes subsistant éventuellement peuvent être recouvertes de façon étanche au gaz.

9. Le dispositif de levage et de basculement selon la revendication 8, **caractérisé en ce que** l'ouverture (9) de plafond est sensiblement rectangulaire et le mât (5) de guidage ou la partie (8) supérieure de mât est disposé(e) dans la région centrale d'un petit côté du rectangle, et
**en ce que** la caisse (16) de mât est disposée dans la région centrale de ce petit côté du rectangle ou s'étend, sous forme de caisse de mât étroite, sur toute la région de ce petit côté du rectangle respectivement jusqu'aux grands côtés opposés du rectangle.

10. Le dispositif de levage et de basculement selon la revendication 8 ou 9, **caractérisé en ce que** la caisse (16) de mât est formée par une charpente en cadre avec une fermeture par les parois (17) latérales, parmi lesquelles au moins la paroi (18) d'entretien peut être ouverte, et par la plaque (19) de couverture, dans lequel des entretoises de soutien peuvent au besoin être reliées directement ou indirectement à la charpente en cadre.

11. Le dispositif de levage et de basculement selon l'une quelconque des revendications 8 à 10, **caractérisé en ce que** la plaque (15) de socle et la paroi (18) d'entretien sont formées ensemble en une seule pièce ou en plusieurs parties.

12. Le dispositif de levage et de basculement selon l'une quelconque des revendications 8 à 11, **caractérisé en ce que** l'ouverture (9) de plafond est située dans un plafond horizontal du récipient de fermentation, en particulier un plafond en béton, ou dans un toit incliné en film, et la caisse (16) de mât est saillante vers le haut au-dessus de l'ouverture (9) de plafond.
